# FASCICULE DE BREVET EUROPEEN

(11) **EP 0 958 501 B1**
(45) Date de publication et mention de la délivrance du brevet: **02.11.2006**
(21) Numéro de dépôt: 98902078.9
(22) Date de dépôt: 14.01.1998
(51) Int. Cl.: G01N 33/68, C07K 14/47, C07K 1/14

(54) **METHODE DE CRIBLAGE DE SUBSTANCES A ACTION THERAPEUTIQUE DANS LE TRAITEMENT DES ENCEPHALOPATHIES SUBAIGUES SPONGIFORMES TRANSMISSIBLES**
VERFAHREN ZUM SCREENING VON SUBSTANZEN MIT THERAPEUTISCHER WIRKUNG BEI DER BEHANDLUNG VON SUBAKUTEN ÜBERTRAGBAREN SPONGIFORMEN ENCEPHALOPATHIES
METHOD FOR SCREENING SUBSTANCES WITH THERAPEUTIC ACTION IN THE TREATMENT OF TRANSMISSIBLE SUBACUTE SPONGIFORM ENCEPHALOPATHY

(30) Priorité: 14.01.1997 FR 9700278
(43) Date de publication de la demande: 24.11.1999
(73) Titulaire: COMMISSARIAT A L'ENERGIE ATOMIQUE, 75015 Paris (FR)
(72) Inventeur: DESLYS, Jean-Philippe, F-78170 La Celle Saint Cloud (FR); BERINGUE, Vincent, F-78850 Thiverval Grignon (FR); LASMEZAS, Corinne, F-75019 Paris (FR)
(74) Mandataire: Goulard, Sophie
(86) Numéro de dépôt international: PCT/FR1998/000063
(87) Numéro de publication internationale: WO 1998/030909

(56) Documents cités:
- WO-A-93/23432
- US-A- 5 276 059
- D. MCKENZIE ET AL.: "Amphotericin B delays both scrapie agent replication and PrP-res accumulation early in infection." JOURNAL OF VIROLOGY., vol. 68, no. 11, novembre 1994, ICAN SOCIETY FOR MICROBIOLOGY US, pages 7534-7536, XP002042242
- S. SAKAGUCHI ET AL.: "Accumulation of proteinase K-resistant prion protein (PrP) is restricted by the expression level of normal PrP in mice inoculated with a mouse-adapted strain of the Creutzfeldt-Jakob disease agent." JOURNAL OF VIROLOGY., vol. 69, no. 12, décembre 1995, ICAN SOCIETY FOR MICROBIOLOGY US, pages 7586-7592, XP002042243
- R. B. PETERSEN ET AL.: "Effect of the D178N mutation and the codon 129 polymorphism on the metabolism of the prion protein." JOURNAL OF BIOLOGICAL CHEMISTRY., vol. 271, no. 21, 24 mai 1996, MD US, pages 12661-12668, XP002042244
- BOLTON ET AL: "Isolation and structural studies of the intact scrapie agent protein" ARCHIVES OF BIOCHEMISTRY AND IBOPHYSICS, vol. 258, no. 2, 1 novembre 1987 (1987-11-01), pages 579-590,
- YOU GENG XI ET AL: "Detection of proteinase resistant protein (PrP) in small brain tissue samples from Creutzfeldt-Jakob disease patients" JOURNAL OF NEUROLOGICAL SCIENCES, vol. 24, 1994, pages 171-173,

## Description

La présente invention est relative à une méthode de criblage de substances, susceptibles d'avoir une action thérapeutique dans le traitement des encéphalopathies subaiguës spongiformes transmissibles (ESST) ou maladies dites à prions, qui comprend une étape d'isolement de la PrPres, à partir de la rate ; la présente invention est également relative à des procédés d'isolement de la PrPres, particulièrement adaptés à ladite méthode de criblage ainsi qu'à leurs applications, notamment dans la détection de la PrPres.

Les encéphalopathies subaiguës spongiformes transmissibles sont provoquées par des agents transmissibles non conventionnels (ANTC), encore appelés prions, dont la nature précise demeure inconnue à ce jour. Les ESST comprennent essentiellement la maladie de Creutzfeldt-Jakob, chez l'homme (MCJ ou CJD pour Creutzfeldt-Jakob *disease*), la tremblante, chez le mouton et la chèvre et l'encéphalopathie spongiforme bovine (ESB ou BSE pour *bovine spongiform encephalopathy*), chez les bovins ; d'autres encéphalopathies ont été mises en évidence chez le vison ou certains animaux sauvages, tels que le cerf et l'élan.

Ces maladies sont d'évolution constamment fatale et il n'existe, à l'heure actuelle, aucun traitement efficace.

Dans les encéphalopathies subaiguës spongiformes transmissibles, il existe une accumulation d'une protéine de l'hôte, la PrP (ou protéine du prion), sous une forme anormale (PrPres), principalement dans le système nerveux central ; la PrPres copurifie avec l'infectiosité et son accumulation précède l'apparition des lésions histologiques. *In vitro,* elle est toxique pour des cultures de neurones.

Deux propriétés biochimiques permettent de distinguer la PrPres de la PrP normale : la PrPres est partiellement résistante aux protéases et est insoluble dans les détergents non-ioniques, tels que le Triton-X100.

L'Article D. McKenzie *et al.,* Journal of Virology, 68, 11, 1994, 7534-7536 est relatif à l'étude de l'action de l'amphotéricine B sur les encéphalopathies subaiguës spongiformes transmissibles (ESST). L'effet de l'amphotéricine B est étudié sur le cerveau de hamsters qui sont sacrifiés 4, 5, 6, 8 et 10 semaines après l'infection.

Le Brevet US-5,276,059 étudie l'action du rouge congo sur l'accumulation de PrPres en utilisant comme modèle des souris infectées par la tremblante. Les rates des souris sont soumises à plusieurs traitements successifs et répétés de sonication de digestion et de centrifugation.

La recherche de nouvelles molécules susceptibles d'être efficaces dans le traitement de ces encéphalopathies se heurte à l'absence aussi bien de modèles *in vitro* performants, qu'à la longueur de la mise en place des modèles expérimentaux *in vivo,* tels que la tremblante expérimentale du hamster (80 à 365 jours) ou la tremblante expérimentale de la souris (180 à 550 jours).

En conséquence, les Inventeurs se sont donné pour but de pourvoir à une méthode de criblage efficace et fiable, qui ne présente pas les inconvénients des modèles expérimentaux actuellement utilisés et qui répond mieux aux besoins de la pratique, notamment en ce que l'évaluation de l'action des substances à tester peut être réalisée en moins de deux mois.

Pour ce faire, les Inventeurs ont trouvé un marqueur fiable et ont mis au point un protocole reproductible.

La présente invention a pour objet une méthode de criblage de substances, susceptibles d'avoir une action thérapeutique dans le traitement des encéphalopathies subaiguës spongiformes transmissibles (ESST ou maladies dites à prions), caractérisée en ce qu'elle comprend les étapes suivantes :
a) inoculation au moment t_{A}, à au moins un animal de laboratoire sélectionné dans le groupe constitué par les rongeurs (de préférence plusieurs, répartis en lots), par toute voie appropriée, d'un agent transmissible non conventionnel (ATNC) ou prion ;
b) administration audit animal de laboratoire, par toute voie appropriée, soit d'une substance à cribler (animal test), soit d'un placebo (animal contrôle négatif), dans un délai compris entre t_{A} 15 jours et t_{C}, correspondant au moment où le taux de PrPres dans la rate dudit animal de laboratoire est maximal ou dans un délai compris entre t_{B}, correspondant au moment de la première détection de PrPres dans la rate dudit animal de laboratoire et t_{C} ; t_{B} étant compris entre t_{A} et t_{A} + 15 et t_{C} étant compris entre t_{A} + 20 et t_{A} + 30, de préférence entre t_{A} + 25 et t_{A} + 30 ;
c) sacrifice des animaux dans un intervalle de temps compris entre t_{B} et t_{C}, de préférence à t_{C} et prélèvement de la rate ; t_{A}, t_{B} et t_{C} étant exprimés en jours ;
d) isolement de la PrPres à partir de chaque rate prélevée, suivant le procédé d'isolement tel que défini ci-après et comprenant l'homogénéisation de la rate, suivie d'une extraction spécifique de la PrPres comprenant une seule étape de séparation, à partir de l'homogénat obtenu et éventuellement la purification de la PrPres ;
e) semi-quantification de la PrPres obtenue à l'étape (d) par détection de ladite PrPres par toute méthode appropriée, produisant un signal spécifique, suivie d'une comparaison du signal obtenu avec une gamme étalon de dilutions d'un contrôle positif constitué d'un homogénat de cerveau d'un animal au stade terminal de la maladie ; et
f) sélection de la substance criblée comme candidat au traitement des encéphalopathies subaiguës spongiformes transmissibles, si le taux de PrPres obtenu dans la rate de l'animal test, à l'étape e) est diminué d'au moins un facteur 2, par rapport au taux obtenu dans les mêmes conditions avec l'animal contrôle négatif.

Les temps t_{A}, t_{B} et t_{C} sont exprimés en jours ; t_{A} = J0.

En effet, les Inventeurs ont trouvé, de manière inattendue, que les substances qui augmentent la survie d'animaux infectés, quelle que soit la voie d'inoculation (périphérique ou intracérébrale), entraînent également un retard dans l'accumulation de la PrPres au niveau de la rate, détectée dans des conditions standardisées.

On entend par conditions standardisées, au sens de la présente invention, des conditions dans lesquelles les paramètres suivants sont sélectionnés :
- ATNC sélectionné,
- voie d'administration de l'ATNC,
- méthode d'isolement de la PrPres à partir de la rate.

Pour une souche sélectionnée dans un animal donné, au stade terminal de la maladie, le titre infectieux est constant.

La détection de la PrPres dans la rate permet d'observer beaucoup plus rapidement les effets des molécules à tester, en particulier l'inhibition de l'accumulation de la PrPres, soit dans les heures qui suivent l'inoculation (capture de l'inoculum par la rate et détection d'un pic de PrPres, entre t_{A} et t_{A} + 1-2 jours), soit entre 5 et 15 jours après l'infection ; t_{B} correspond aussi bien à la détection du pic au moment de la capture de l'inoculum qu'à la détection de la PrPres néosynthétisée ; c'est la raison pour laquelle t_{B} est compris entre t_{A} et t_{A} + 15 ; ces valeurs de t_{B} et de t_{C} peuvent varier dans les fourchette définies ci-dessus, en fonction de l'ATNC et de l'animal de laboratoire (souris) sélectionnés ; par exemple, lorsque l'ATNC correspond à la souche murine C506M3 inoculée par voie intrapéritonéale, chez la souris C57BL/6, la PrPres néosynthétisée peut être détectée dans 100 % des cas, dès le 5^{ème} jour post-infection (p.i.) (t_{B}) et un plateau est observé à partir du 30^{ème} jour p.i. (t_{C}).

Une telle méthode permet donc de sélectionner des molécules capables d'empêcher l'accumulation de PrPres ; de telles molécules sont considérées comme pouvant présenter une action thérapeutique dans le traitement des ESST.

Conformément à l'invention :
*** à l'étape a) :**
   - l'ATNC correspond à une souche stabilisée chez l'animal hôte, c'est-à-dire qui présente des caractéristiques stables chez cet animal hôte après plusieurs passages et notamment les caractéristiques suivantes : délai d'apparition de la maladie identique et profil lésionnel identique au cours des passages chez tous les animaux (degré de vacuolisation de différentes parties du cerveau) ; il correspond à n'importe quelle souche stabilisée dans les conditions précisées ci-dessus, induisant une accumulation précoce de PrPres dans la rate de l'animal hôte, telle que des souches de tremblante ou des souches d'encéphalopathie bovine, notamment les souches de tremblante dénommées Chandler, ME7, 139A (M.E. Bruce et al., *Scrapie strain variation and its implication* dans *Current topics in Microbiology and Immunology : Transmissible Spongifom Encephalopathies, Scrapie, BSE and related Disorders,* 1991, **172**, 125-138), C506M3 (C.I. Lasmézas et al., J. Gen. Virol., 1996, 77, 1601-1609) ou 263K (R.H. Kimberlin et al., J. Gen. Virol., 1977, **34**, 295-304 et 1978, **39**, 487-496) ou les souches de BSE dénommées 4PB1 (C.I. Lasmézas et al., 1996, précité) et 301V (C.F. Farquhar et al., J. Gen. Virol., 1996, 77, 1941-1946) ;
   - ledit ATNC est, de préférence administré dans un tampon adapté à la voie d'administration sélectionnée, sous la forme soit d'un homogénat brut de tissu, de préférence de cerveau, soit d'un culot de PrPres, obtenu par centrifugation convenable, à partir d'un homogénat brut de tissu, de préférence de cerveau ;
   - ledit ATNC peut être administré par n'importe quelle voie (voie orale, voie parentérale), de préférence par voie intrapéritonéale, à une dose correspondant à un inoculum d'ATNC, compris entre 0,001 % et 10 % (poids/volume) (DL₅₀ comprise entre 10³ et 10⁷) ;
   - ledit animal de laboratoire est de préférence un rongeur (souris ou hamster, par exemple).
*** à l'étape b) :**
   - la substance à cribler est administrée par voie orale ou parentérale ;
   - si le traitement est commencé entre t_{B} et t_{C}, (c'est-à-dire lorsque la PrPres dans la rate est constamment détectable), le modèle selon l'invention permet d'étudier uniquement l'action de la substance à cribler sur l'ATNC inoculé en cours de réplication au niveau des sites de réplication (cellules cibles), alors que si elle est administrée avant t_{B}, par exemple à t_{A}, le modèle selon l'invention permet d'étudier en plus l'action de la substance à cribler, avant que l'ATNC n'ait atteint ses cellules cibles dans la rate.
*** à l'étape c) :**
   - le sacrifice de l'animal est réalisé à t_{C}.
*** à l'étape d) :**
   - selon la séquence d'étapes sélectionnées parmi les techniques d'isolement des protéines connues, à savoir les méthodes basées sur la taille moléculaire, telle que la centrifugation, les méthodes basées sur les différences de solubilité telles que la dissolution par les sels (*salting-in*) et le relargage (*salting-out*) ou le fractionnement par des solvants ou les méthodes basées sur la charge électrique, le degré de purification et le rendement seront différents. Dans le cadre de la présente invention, il est nécessaire de sélectionner une méthode fiable et sensible, permettant d'obtenir un seuil de détection tel que le rapport taux maximal détectable dans la rate/valeur seuil (*cut off*) soit le plus élevé possible, de préférence supérieur à 2 ou tel que lorsqu'on l'on réalise une dilution au ½ de l'échantillon final obtenu, on obtient encore un signal de détection ;
   - des séquences d'étapes préférées sont décrites ci-après : elles ont l'avantage, sur les procédés d'isolement antérieurement décrits, de présenter une grande fiabilité et une grande sensibilité, du fait que l'extraction proprement dite ne comprend qu'une seule étape de séparation et du fait de la sélection particulière de la séquence d'étapes, alors que dans les procédés antérieurement décrits (R.E. Race et al., J. Gen. Virol., 1992, **73**, 3319-3323 ; Doi et al., J. Gen. Virol., 1988, **69**, 955-960 ; T. Muramoto et al., Am. J. Pathol., 1993, **143,** 5 1470-1479; Farquhar C.F. et al., Gen. Virol., 1994, **75**, 495-504 et J. Gen. Virol., 1996, **77**, 1941-1946), l'extraction comprend plusieurs étapes de séparation et conduit à une imprécision pour ce qui concerne la quantification et/ou ces procédés présentent une sensibilité insuffisante pour obtenir un seuil de détection et une quantification fines et notamment pour effectivement détecter une variation importante du taux de PrPres.
*** à l'étape e) :**
   - la PrPres est notamment détectée par immunoessai (Western blot par exemple).
      La présente invention a également pour objet un procédé d'isolement de la PrPres, dans lequel l'extraction comprend une seule étape pour la séparation de la PrPres, et ne nécessite pas d'ultracentrifugation; un tel procédé d'isolement de la PrPres, à partir d'un organe ou d'un tissu, notamment la rate ou le cerveau est caractérisé en ce qu'il consiste essentiellement en les étapes suivantes :
      (i) homogénéisation d'organe ou de tissu, prélevés après le sacrifice de l'animal, par broyage mécanique dans un tampon d'homogénéisation, puis addition à l'homogénat obtenu, d'un sel ayant une force ionique élevée et apte à favoriser l'agrégation de la PrPres, tel que du NaCl à 10-30 %, dans un rapport 1:1 (v/v), suivi d'un calibrage de l'homogénat, pour l'obtention d'un homogénat comprenant, en poids/volume, de 5 à 50 % dudit organe ou tissu ;
      (ii) extraction spécifique de la PrPres par traitement de l'homogénat obtenu à l'étape (i) par incubation de la suspension obtenue avec une solution comprenant une protéase et un détergent anionique apte à favoriser l'agrégation de la PrPres, tel que du sarkosyl à 10-30 % et une seule étape de séparation de la PrPres, par centrifugation à 25 000-60 000 g, par exemple à 25 000-30 000 g pendant 1-2 heures, de préférence à 16-22°C, de la suspension obtenue, déposée sur un coussin de tampon ayant une densité comprise entre 1,02 et 1,08, à 20°C et récupération du culot de centrifugation comprenant ladite PrPres ; et, si nécessaire
      (iii) purification de la PrPres par mise en suspension du culot de centrifugation obtenu en (ii) dans un tampon de Laemmli comprenant du SDS à 1-5 %, incubation dans ce tampon à 100°C pendant 2-10 minutes et centrifugation à 12 000-15 000 g pendant 10-15 minutes à 16-22°C.

Ladite PrPres ainsi purifiée peut ensuite être séparée par toute technique appropriée telle qu'une électrophorèse (électrophorèse sur gel de polyacrylamide, par exemple) ou une immunocapture, à partir du surnageant de centrifugation.

Conformément à ce procédé, le tampon d'homogénéisation de l'étape (i) est notamment un tampon neutre tel que de l'eau ou un tampon isotonique tel que du glucose à 5% ;

Également conformément à l'invention :
- lors de l'étape (ii) d'extraction, la solution mise en oeuvre pour l'extraction comprend un détergent anionique, apte à favoriser l'agrégation de la PrPres et un détergent ayant des propriétés de renaturation des protéines, tel qu'un détergent zwitterionique, comme une sulfobétaïne, de préférence la sulfobétaïne SB 3-14 à 1-2 %, dans un rapport 1:1 (v/v) ;
- lors de l'étape (ii) d'extraction, mais préalablement à la centrifugation, au moins un inhibiteur des protéases est ajouté ;
- la centrifugation selon l'étape (ii) d'extraction est, de préférence, réalisée après dépôt de la suspension contenant la PrPres sur un coussin de saccharose à 6-20 % ou un coussin de saccharose à 6-20 % et d'une sulfobétaïne.

La PrPres peut ensuite être détectée par toute méthode spécifique appropriée.

De manière surprenante, ces procédés d'isolement de la PrPres, à partir de la rate, comprenant une extraction en une seule étape, n'entraînent pas de perte cumulative de PrPres et sont directement utilisables sans modification, pour extraire la PrPres de n'importe quel autre tissu.
- la figure 1 illustre le protocole mis en oeuvre dans une méthode de criblage;
- la figure 2 représente un gel de polyacrylamide montrant l'inhibition de l'accumulation de la PrPres dans les rates de souris infectées par la souche C506M3 et traitées par l'amphotéricine B (AmB) (l'échelle des poids moléculaires a été établie avec des marqueurs précolorés Amersham) ;
- la figure 3, sous la forme d'un histogramme, illustre l'inhibition de l'accumulation de la PrPres, chez la même souris, après traitement par l'amphotéricine B ou l'ABLC® (*AmB Lipid Complex*)*,* par rapport à un animal contrôle négatif, traité par un placebo et dans lequel il n'y a pas d'inhibition de ladite accumulation ;
- les figures 4 et 5 illustrent la cinétique d'accumulation de la PrPres dans la rate de souris C57BL/6 inoculées i.p. par la souche C506M3 (0-28 jours post-inoculation) ;
- la figure 6 illustre le rôle de la composition du tampon d'extraction, dans le rendement de purification de la PrPres ;
- la figure 7 illustre le protocole de traitement mis en oeuvre pour tester le sulfate de dextran (DS500) ;
- la figure 8 illustre l'accumulation de la PrPres dans des rates de souris C57BL/6, infectées par voie intrapéritonéale, par la souche C506M3 et traitées 2h avant l'inoculation par le sulfate de dextran DS500.

Il doit être bien entendu, toutefois, que ces exemples sont donnés uniquement à titre d'illustration de l'objet de l'invention, dont ils ne constituent en aucune manière une limitation.

### EXEMPLE 1 : Étude de l'accumulation de la PrPres dans des rates de souris C57BL/6, infectées par voie intrapéritonéale (ip) par la souche C506M3 et traitées pendant 1 ou 2 semaines (6 jours/semaine), à partir de t_{A}+15 après l'inoculation, par l'amphotéricine B (AmB) et ses dérivés ; isolement de la PrPres présente dans la rate par le procédé d'isolement comportant une ultracentrifugation, tel que décrit ci-dessus.

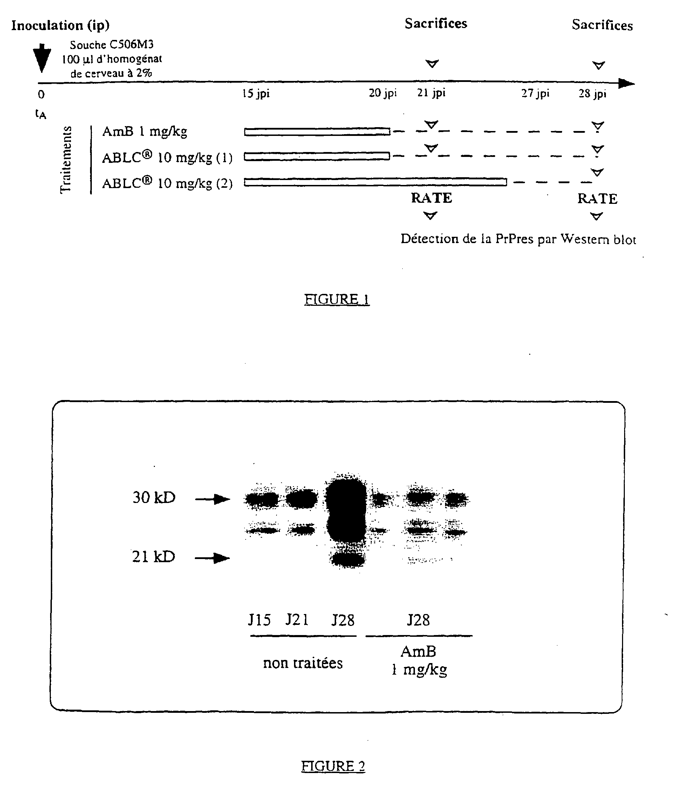

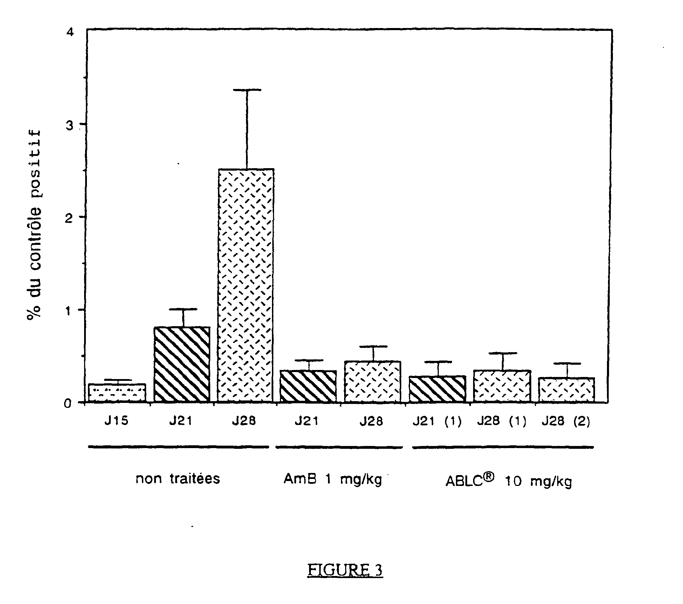

- étape a) de la méthode de criblage : inoculation
   à t_{A}, des souris C57BL/6 sont inoculées par voie intrapéritonéale avec 100 µl d'homogénat de cerveau à 2 % dans du glucosé 5 %, d'une souris infectée au stade terminal de la tremblante expérimentale (souche C506M3).
- étape b) de la méthode de criblage : administration d'une substance susceptible d'avoir une action thérapeutique ou administration d'un placebo
   à t_{A}+15 jours (→ délai compris entre t_{B} et t_{C}), les souris C57BL/6 sont réparties en différents lots et sont traitées :
   - soit avec de l'amphotéricine B, à raison de 1 mg/kg (AmB),
   - soit avec de l'ABLC^{®}, à raison de 10 mg/kg, pendant 6 jours (1) ou 12 jours (2), conformément à la figure 1,
   - soit avec un placebo.
- étape c) de la méthode de criblage : sacrifice des animaux.
   À t_{A}+21 jours (→ délai compris entre t_{B} et t_{C}) ou à t_{A}+28 jours (→ à t_{C}), les souris sont sacrifiées par rupture des vertèbres cervicales ; les rates sont immédiatement prélevées, conformément à la figure 1, et soit stockées à -80°C, soit utilisées extemporanément.
- étape d) de la méthode de criblage : isolement de la PrPres
   Les rates prélevées sont broyées et homogénéisées à 10 % (poids/volume) dans une solution de glucose à 5 %. L'homogénat obtenu est calibré, par passage dans une seringue convenable.
   L'homogénat à 10 % (200 µl) est ensuite traité à la protéinase K (10 µg/ml), à 37°C, pendant une heure ; la digestion est bloquée à l'aide de fluorure de phénylméthylsulfonyle (PMSF) 5 mM. Après addition de sarkosyl à 20 % dans du Tris pH 7,4 10 mM, les échantillons sont incubés pendant 15 minutes à température ambiante. Ils sont ensuite centrifugés à 245 000 g pendant 4 heures à 20°C, sur un coussin de saccharose à 10 % (100-300 µl) (ultracentrifugeuse Beckman TL100).
   Les culots sont remis en suspension dans un tampon de Laemmli, incubés 5 minutes à 100°C, puis les échantillons obtenus sont soumis à une centrifugation à 15 000 g, pendant 1 minutes à 16°C.
- étape e) de la méthode de criblage : détection de la PrPres dans les échantillons.
   Les échantillons obtenus sont utilisés pour réaliser une électrophorèse SDS-PAGE (gel de polyacrylamide à 12 % chargé avec l'équivalent de 10 mg de rate) et transféré sur membrane de nitrocellulose, dans les conditions décrites par Towbin et al. (Proc. Natl. Acad. Sci. USA, 1979, **76**, 4350-4354) ou par C.I. Lasmézas et al. (J. Gen. Virol., 1996, précité). L'immunodétection de la PrPres a été réalisée avec l'antisérum 007 JB (R. Demaimay et al., Journal of Virology, 1997, 71, 12, 9685-9689), dirigé contre le peptide 90-108 de la PrP murine au 1/2500) et des Ig de chèvre anti-lapin conjuguées à de la peroxydase (1/2500). L'immunoréactivité est révélée par chimioluminescence (ECL, Amersham), quantifiée et visualisée sur films autoradiographiques, comme illustré sur la figure 2 , pour les animaux non traités et les animaux traités pendant 6 jours par l'AmB à 1 mg/kg et sacrifiés à t_{A}+28 jours, c'est-à-dire une semaine après la fin du traitement.
   Les anticorps sont obtenus par couplage dudit peptide (Néosystem, Strasbourg) à la KLH, puis injection sous-cutanée dans la région dorsale de lapins « Nouvelle-Zélande » d'une émulsion comprenant ledit peptide couplé et de l'adjuvant complet de Freund (R. Demaimay et al., Journal of Virology, 1997, 71, 12, 9685-9689).

* étape f) de la méthode de criblage : sélection de la substance criblée
   La figure 3 illustre les résultats obtenus pour les animaux non traités, les animaux traités par l'AmB, 1 mg/kg sacrifiés à t_{A}+21 ou à t_{A}+28 et les animaux traités par l'ABLC® 6 jours (1) ou 12 jours (2) et sacrifiés à t_{A}+21 ou à t_{A}+28 : aussi bien pour les animaux traités par l'AmB que par l'ABLC^{®}, on observe une inhibition significative de l'accumulation de PrPres ; pour construire l'histogramme, les quantités de PrPres détectées dans la rate sont rapportées à une gamme linéaire de dilutions de PrPres purifiée selon le même procédé que celui décrit ci-dessus, à partir d'un homogénat de cerveau d'animaux au stade terminal de la maladie (contrôle positif).

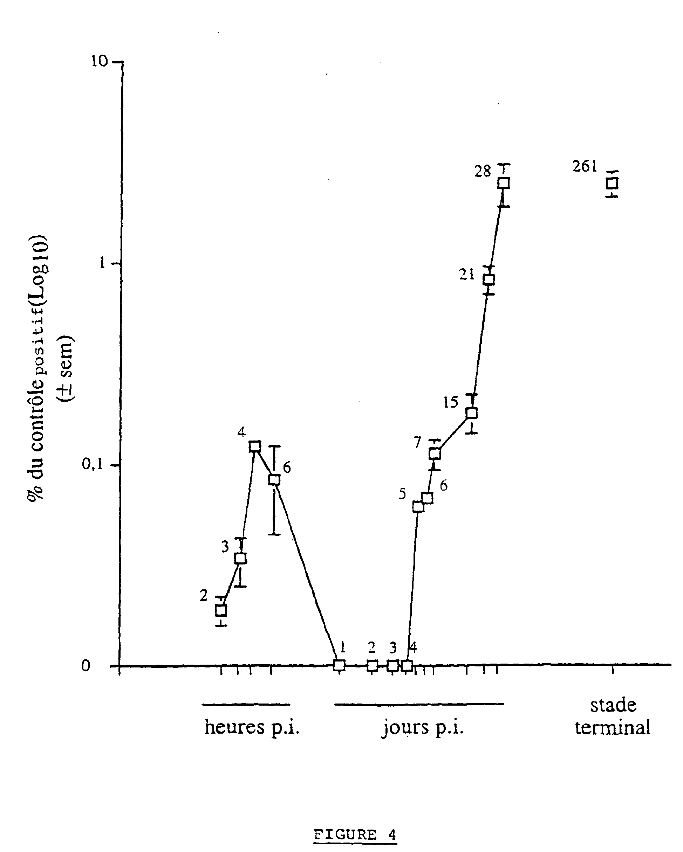

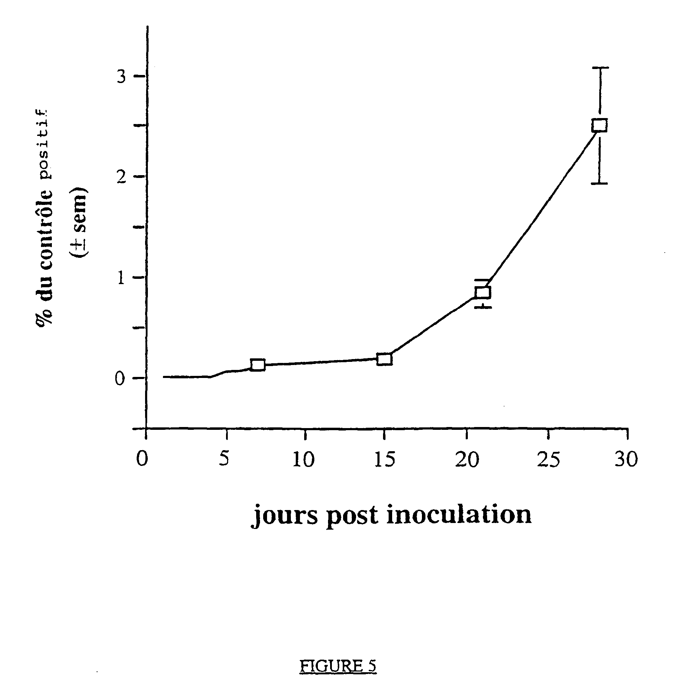

   Les figures 4 et 5 illustrent la cinétique d'accumulation de la PrPres dans la rate de souris C57BL/6, inoculées par la souche C506M3 à t_{A}, dans les mêmes conditions que ci-dessus, pendant 28 jours et non traitées : on observe une augmentation progressive jusqu'à t_{A}+30 (→ à t_{C}) ; un plateau est observé à partir de t_{A}+30.

### EXEMPLE 2 : Étude de l'accumulation de la PrPres dans des rates de souris C57BL/6, infectées par voie intrapéritonéale (ip) par la souche C506M3 et traitées pendant 1 ou 2 semaines (6 jours/semaine), à partir de t_{A}+15 après l'inoculation, par l'amphotéricine B (AmB) et ses dérivés ; isolement de la PrPres par le procédé ne comportant pas d'ultracentrifugation.

Les étapes a), b), c), e) et f) sont identiques à celles de l'exemple 1.

L'étape d) d'isolement de la PrPres à partir des rates de souris est réalisée comme suit :
Les rates prélevées sont broyées et homogénéisées à 20 % (poids/volume) dans une solution contenant du glucose à 5 % ; on ajoute à 200 µl d'homogénat, 200 µl de NaCl à 20 % (1:1, v/v). L'homogénat obtenu est calibré, par passage dans une seringue convenable.
On ajoute à 200 µl d'homogénat à 20 %, 200 µl de détergent (sarkosyl à 20 % et sulfobétaïne à 2 % (SB3.14 Calbiochem)) et de la protéinase K à 10 µg/ml, puis on incube à 37°C, pendant une heure.
Les échantillons sont ensuite centrifugés à 30 000 g pendant 2 heures à 22°C, sur 200 µl d'un coussin comprenant du saccharose à 10% et de la sulfobétaïne à 0,1%, en concentrations finales (rotor Eppendorf ; centrifugeuse ALC 4239R).

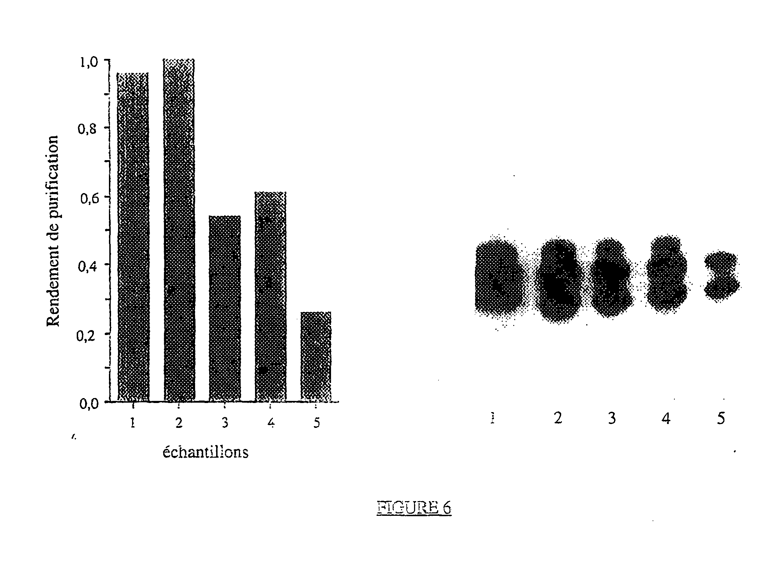

La figure 6 illustre les rendements de purification obtenus avec différentes compositions de tampons d'extraction (représentation sous la forme d'un histogramme et d'un Western blot) : 1 : témoin de rendement (homogénat total) ; 2 : sarkosyl à 10 %/NaCl à 10 %/Tris 10 mM/SB3-14 à 1 % ; 3 : sarkosyl à 10 %/NaCl à 10 %/Tris 10 mM ; 4 : sarkosyl à 10 %/NaCl à 10 % ; 5 : sarkosyl à 10 %.

Les culots sont remis en suspension dans un tampon de Laemmli, incubés 5 minutes à 100°C, puis les échantillons obtenus sont soumis à une deuxième centrifugation à 15000 g, pendant 15 minutes à 16°C.

### EXEMPLE 3 : Étude de l'accumulation de la PrPres dans des rates de souris C57BL/6 infectées par voie intrapéritonéale par la souche C506M3 et traités à t_{A-}2 heures par le sulfate de dextran (DS500).

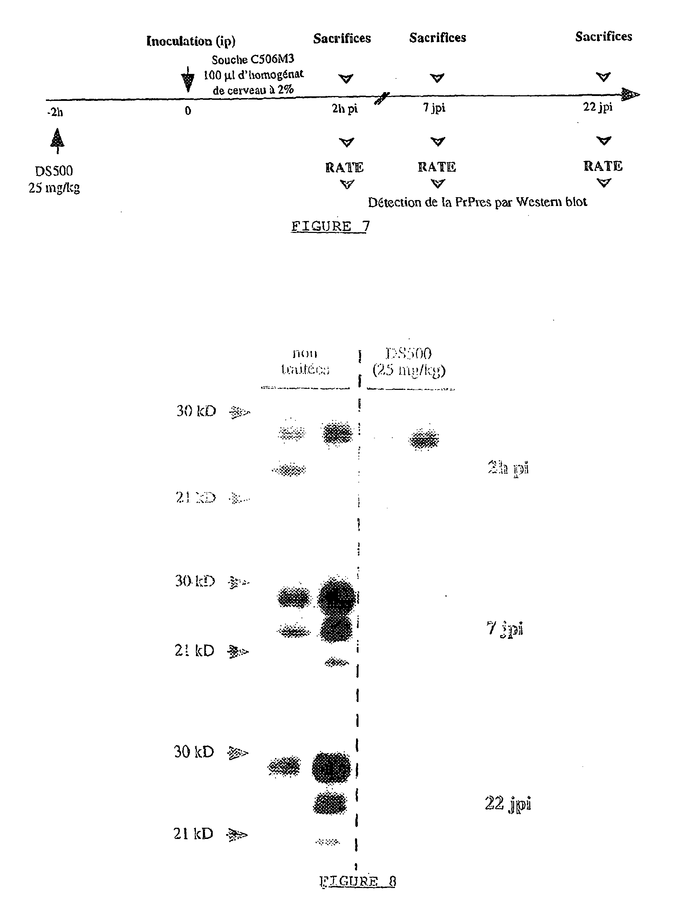

Le protocole de traitement est résumé à la figure 7.
- étape b) de la méthode de criblage : administration d'une substance susceptible d'avoir une action thérapeutique
   A t_{A}- 2 heures (c'est-à-dire 2 heures avant l'inoculation de la souche infectante), des souris C57BL/6 sont réparties en différents lots :
   - souris non traitées
   - et souris traitées avec du sulfate de dextran (DS500) à 25 mg/kg (injection unique à t_{A}-2 heures).
- étape a) de la méthode de criblage : inoculation
   à t_{A}, les souris C57BL/6 sont inoculées par voie intrapéritonéale avec 100 µl d'homogénat de cerveau à 2 % dans du glucosé 5 %, d'une souris infectée au stade terminal de la tremblante expérimentale [souche C506M3].
- étape c) de la méthode de criblage : sacrifice des animaux.
   À t_{A}+ 2 heures, t_{A}+7 jours et t_{A}+22 jours, les souris sont sacrifiées par rupture des vertèbres cervicales ; les rates sont immédiatement prélevées.
- étape d) de la méthode de criblage : isolement de la PrPres
   identique à l'étape d) de l'exemple 2.
- étape e) de la méthode de criblage selon l'invention : détection de la PrPres dans les échantillons.
   Les échantillons obtenus sont utilisés pour réaliser une électrophorèse SDS-PAGE (gel de polyacrylamide à 12 % chargé avec l'équivalent de 40 mg de rate pour 2 h et 7 jours et de 10 mg de rate pour 22 jours) et transféré sur membrane de nitrocellulose, dans les conditions décrites par Towbin et al. (Proc. Natl. Acad. Sci. USA, 1979, 76, 4350-4354) ou par C.I. Lasmézas et al. (J. Gen. Virol., 1996, précité). L'immunodétection de la PrPres a été réalisée avec l'antisérum 007JB (R. Demaimay et al., J. Virol, 1997, **71**, 12, 9685-9689) au 1/5 000^{ème}, et des Ig de chèvre anti-lapin conjuguées à de la peroxydase (1/2500). L'immunoréactivité est révélée par chimioluminescence (ECL, Amersham), quantifiée et visualisée sur films autoradiographiques, comme illustré sur la figure 8 , pour les animaux non traités et les animaux traités 2 h avant l'inoculation par le DS500 et sacrifiés à t_{A} + 2 h, t_{A} + 7 jours et t_{A}* + 22 jours.

* étape f) de la méthode de criblage selon l'invention : sélection de la substance criblée
   La figure 8 illustre les résultats obtenus ; on observe une inhibition significative de l'accumulation de PrPres, chez les animaux traités.
   Ainsi que cela ressort de ce qui précède, l'invention ne se limite nullement à ceux de ses modes de mise en oeuvre, de réalisation et d'application qui viennent d'être décrits de façon plus explicite ;

## Revendications

1. Procédé d'isolement de la PrPres, à partir d'un organe ou d'un tissu, **caractérisé en ce qu'**il consiste essentiellement en les étapes suivantes :
(i) homogénéisation d'organe ou de tissu, prélevés après le sacrifice de l'animal, par broyage mécanique dans un tampon d'homogénéisation, puis addition à l'homogénat obtenu, d'un sel ayant une force ionique élevée et apte à favoriser l'agrégation de la PrPres dans un rapport 1 : 1 (v/v), suivi d'un calibrage de l'homogénat, pour l'obtention d'un homogénat comprenant, en poids/volume, de 5 à 50 % dudit organe ou tissu ;
(ii) extraction spécifique de la PrPres par traitement de l'homogénat obtenu à l'étape (i) par incubation de la suspension obtenue avec une solution comprenant une protéase et un détergent anionique apte à favoriser l'agrégation de la PrPres et une seule étape de séparation de la PrPres, par centrifugation à 25 000-60 000 g. de la suspension obtenue, déposée sur un coussin de tampon ayant une densité comprise entre 1,02 et 1,08, à 20°C et récupération du culot de centrifugation comprenant ladite PrPres.

2. Procédé selon la revendication 1, **caractérisé en ce qu'**il comprend en outre une étape de purification de la PrPres par mise en suspension du culot de centrifugation obtenu en (ii) dans un tampon de Laemmli comprenant du SDS à 1-5 %, incubation dans ce tampon à 100°C pendant 2-10 minutes et centrifugation à 12 000-15 000 g pendant 10-15 minutes à 16-22°C.

3. Procédé selon la revendication 1 ou la revendication 2, **caractérisé en ce que** le tampon d'homogénéisation de l'étape (i) est un tampon neutre ou un tampon isotonique.

4. Procédé selon la revendication 3, **caractérisé en ce que** le tampon d'homogénéisation est choisi parmi : l'eau et le glucose à 5 %.

5. Procédé selon l'une quelconque des revendications 1 à 4, **caractérisé en ce qu'**à l'étape (ii), préalablement à la centrifugation, au moins un inhibiteur des protéases est ajouté.

6. Procédé selon l'une quelconque des revendication 1 à 5, **caractérisé en ce qu'**à l'étape (ii), la centrifugation est réalisée après dépôt de la suspension contenant la PrPres sur un coussin de saccharose à 6-20 %.

7. Procédé selon l'une quelconque des revendications 1 à 6, **caractérisé en ce que** lors de l'étape (ii) d'extraction la solution mise en oeuvre pour l'extraction comprend un détergent anionique apte à favoriser l'agrégation de la PrPres et un détergent zwitterionique, tel qu'une sulfobétaïne dans un rapport 1 : 1 (v/v).

8. Procédé selon l'une quelconque des revendications 1 à 7, **caractérisé en ce qu'**à l'étape (ii) d'extraction, la centrifugation est réalisée après dépôt de la suspension contenant la PrPres, sur un coussin comprenant en mélange du saccharose à 6-20 % et une sulfobétaïne.

9. Procédé selon l'une quelconque des revendications 1 à 8, **caractérisé en ce que** l'organe ou tissu est sélectionné parmi la rate et le cerveau.

10. Procédé selon l'une quelconque des revendications 1 à 9, **caractérisé en ce qu'**à l'étape (ii) la centrifugation est faite à 25 000-30 000 g pendant 1 à 2 heures à une température de 16 à 22°C.

11. Méthode de criblage de substances, susceptibles d'avoir une action thérapeutique dans le traitement des encéphalopathies subaiguës spongiformes transmissibles (ESST), **caractérisée en ce qu'**elle comprend les étapes suivantes :
a) inoculation au moment t_{A}, à au moins un animal de laboratoire sélectionné dans le groupe constitué par les rongeurs, par toute voie appropriée, d'un agent transmissible non conventionnel (ATNC) ;
b) administration audit animal de laboratoire, par toute voie appropriée, soit d'une substance à cribler (animal test), soit d'un placebo (animal contrôle négatif), dans un délai compris entre t_{A}-15 jours et t_{C}, correspondant au moment où le taux de PrPres dans la rate dudit animal de laboratoire est maximal ou dans un délai compris entre t_{B}, correspondant au moment de la première détection de PrPres dans la rate dudit animal de laboratoire et t_{C} ; t_{B} étant compris entre t_{A} et t_{A} + 15 et t_{C} étant compris entre t_{A} + 25 et t_{A} + 30 ;
c) sacrifice des animaux dans un intervalle de temps compris entre t_{B} et t_{C} et prélèvement de la rate, t_{A}, t_{B} et t_{C} étant exprimés en jours ;
d) isolement de la PrPres à partir de chaque rate prélevée suivant un procédé d'isolement selon l'une quelconque des revendications 1 à 10 ;
e) semi-quantification de la PrPres obtenue à l'étape (d) par détection de ladite PrPres par toute méthode appropriée, produisant un signal spécifique, suivie d'une comparaison du signal obtenu avec une gamme étalon de dilutions d'un contrôle positif constitué d'un homogénat de cerveau d'un animal au stade terminal de la maladie ; et
f) sélection de la substance criblée comme candidat au traitement des encéphalopathies subaiguës spongiformes transmissibles, si le taux de PrPres obtenu dans la rate de l'animal test, à l'étape e) est diminué d'au moins un facteur 2, par rapport au taux obtenu dans les mêmes conditions avec l'animal contrôle négatif.

12. Méthode de criblage selon la revendication 11, **caractérisée en ce qu'**à l'étape a), ledit ATNC est administré dans un tampon adapté à la voie d'administration sélectionnée, sous la forme soit d'un homogénat brut de tissu soit d'un culot de PrPres, obtenu par centrifugation convenable, à partir d'un homogénat brut de tissu.

13. Procédé selon la revendication 12, **caractérisé en ce que** l'homogénat brut de tissu est un homogénat de cerveau.

14. Méthode de criblage selon l'une quelconque des revendications 11 à 13 **caractérisée en ce qu'**à l'étape a), ledit ATNC est administré par voie intrapéritonéale, à une dose correspondant à un inoculum d'ATNC, compris entre 0,001 % et 10 % en poids/volume, la DL₅₀ étant comprise entre 10³ et 10⁷.

15. Méthode de criblage selon l'une quelconque des revendications 11 à 14 **caractérisée en ce qu'**à l'étape d), ladite méthode d'isolement de la PrPres comprend une séparation en une seule étape.

16. . Méthode de criblage selon l'une quelconque des revendications 11 à 15 **caractérisée en ce qu'**à l'étape e), la PrPres est détectée par immunoessai.

17. Méthode selon l'une quelconque des revendications 11 à 16 **caractérisée en ce qu'**à l'étape c) le sacrifice est réalisé à t_{C}.

## Claims

1. Method for isolating PrPres from an organ or a tissue, **characterized in that** it consists essentially of the following steps:
(i) homogenization of an organ or tissue, taken after the animal has been sacrificed, by mechanical grinding in a homogenizing buffer, and then addition to the homogenate obtained of a salt with a high ionic strength, able to promote aggregation of the PrPres, in a 1:1 (v/v) ratio, followed by calibration of the homogenate so as to obtain a homogenate comprising, by weight/volume, from 5 to 50% of said organ or tissue;
(ii) specific extraction of the PrPres by treating the homogenate obtained in step (i) by incubating the suspension obtained with a solution comprising a protease and an anionic detergent able to promote aggregation of the PrPres, and separating the PrPres in a single step, by centrifugation at 25 000-60 000 g of the suspension obtained, deposited onto a cushion of buffer having a density of between 1.02 and 1.08 at 20°C, and recovering of the centrifugation pellet comprising said PrPres.

2. Method according to Claim 1, **characterized in that** it also comprises a step for purifying the PrPres by suspending the centrifugation pellet obtained in (ii) in a Laemmli buffer comprising 1-5% SDS, incubating in this buffer at 100°C for 2-10 minutes and centrifuging at 12 000-15 000 g for 10-15 minutes at 16-22°C.

3. Method according to Claim 1 or Claim 2, **characterized in that** the homogenizing buffer of step (i) is in particular a neutral buffer or an isotonic buffer.

4. Method according to Claim 3, **characterized in that** the homogenizing buffer is chosen from water and 5% glucose.

5. Method according to any one of Claims 1 to 4, **characterized in that**, in step (ii), prior to the centrifugation, at least one protease inhibitor is added.

6. Method according to any one of Claims 1 to 5, **characterized in that**, in step (ii), the centrifugation is carried out after the suspension containing the PrPres has been deposited onto a 6-20% sucrose cushion.

7. Method according to any one of Claims 1 to 6, **characterized in that**, in extraction step (ii), the solution used for the extraction comprises an anionic detergent able to promote aggregation of the PrPres and a zwitterionic detergent, such as a sulfobetaine, in a 1:1 (v/v) ratio.

8. Method according to any one of Claims 1 to 7, **characterized in that**, in extraction step (ii), the centrifugation is carried out after the suspension containing the PrPres has been deposited onto a cushion comprising a mixture of 6-20% sucrose and a sulfobetaine.

9. Method according to any one of Claims 1 to 8, **characterized in that** the organ or tissue is selected from the spleen and the brain.

10. Method according to any one of Claims 1 to 9, **characterized in that**, in step (ii), centrifugation is carried out at 25 000 - 30 000 g for 1 to 2 hours at a temperature of 16 to 22°C.

11. Method for screening substances liable to have a therapeutic action in the treatment of transmissible subacute spongiform encephalopathies (TSSEs), **characterized in that** it comprises the following steps:
a) inoculation, at time t_{A}, of at least one laboratory animal selected from the group consisting of rodents, via any suitable route, with an unconventional transmissible agent (UTA);
b) administration to said laboratory animal, via any suitable route, either of a substance to be screened (test animal) or of a placebo (negative control animal), within a period of time between t_{A} - 15 days and t_{C}, which corresponds to the time at which the PrPres level in the spleen of said laboratory animal is at a maximum, or within a period of time between t_{B}, which corresponds to the time of the first detection of PrPres in the spleen of said laboratory animal, and t_{C}; t_{B} being between t_{A} and t_{A} + 15 and t_{C} being between t_{A} + 25 and t_{A} + 30;
c) sacrifice of the animals within a period of time between t_{B} and t_{C} and removal of the spleen, t_{A}, t_{B} and t_{C} being expressed in days;
d) isolation of the PrPres from each removed spleen, according to an isolation method according to any one of Claims 1 to 10;
e) semi-quantification of the PrPres obtained in step (d), by detection of said PrPres using any suitable method producing a specific signal, followed by comparison of the signal obtained with a standard range of dilutions of a positive control consisting of a brain homogenate from an animal at the terminal stage of the disease; and
f) selection of the substance screened as a candidate for the treatment of transmissible subacute spongiform encephalopathies, if the level of PrPres obtained in the spleen of the test animal, in step e), is decreased at least 2-fold compared to the level obtained under the same conditions with the negative control animal.

12. Screening method according to Claim 11, **characterized in that**, in step a), said UTA is administered in a buffer suitable for the route of administration selected, in the form either of a crude tissue homogenate or of a PrPres pellet obtained, by suitable centrifugation, from a crude tissue homogenate.

13. Method according to Claim 12, **characterized in that** the crude tissue homogenate is a brain homogenate.

14. Screening method according to any one of Claims 11 to 13, **characterized in that**, in step a), said UTA is administered intraperitoneally at a dose corresponding to a UTA inoculum of between 0.001% and 10% by weight/volume, the LD₅₀ being between 10³ and 10⁷.

15. Screening method according to any one of Claims 11 to 14, **characterized in that**, in step d), said method for isolating PrPres comprises separation in a single step.

16. Screening method according to any one of Claims 11 to 14, **characterized in that**, in step e), the PrPres is detected by immunoassay.

17. Method according to any one of Claims 11 to 16, **characterized in that**, in step c), sacrificing is carried out at t_{C}.

## Patentansprüche

1. Verfahren zur Isolierung von PrPres aus einem Organ oder einem Gewebe, **dadurch gekennzeichnet, dass** es im Wesentlichen aus den folgenden Stufen besteht:
(i) Homogenisierung eines Organs oder Gewebes, das nach Töten des Tieres entnommen worden war, durch mechanische Zerkleinerung in einem Homogenisierungspuffer, dann Versetzen des erhaltenen Homogenisats mit einem Salz, das erhöhte Ionenstärke hat und geeignet ist, die Aggregation des PrPres zu fördern, in einem Verhältnis von 1:1 (V/V), gefolgt von einem Einstellen des Homogenisats, um ein Homogenisat zu erhalten, das, als Gewicht/Volumen, 5 bis 50 % des Organs oder Gewebes enthält;
(ii) Spezifische Extraktion des PrPres durch Behandlung des in Stufe (i) erhaltenen Homogenisats durch Inkubation der erhaltenen Suspension mit einer Lösung, die eine Protease und ein anionisches Detergens, geeignet die Aggregation des PrPres zu begünstigen, enthält, und eine einzige Stufe der Abtrennung des PrPres durch Zentrifugation der erhaltenen Suspension, abgeschieden auf einem Pufferkissen mit einer Dichte zwischen 1,02 und 1,08, bei 20°C mit 25.000 bis 60.000 g und Gewinnung des Zentrifugationsrückstands, der das PrPres enthält.

2. Verfahren nach Anspruch 1, **dadurch gekennzeichnet, dass** es außerdem eine Stufe der Reinigung des PrPres durch in Suspension Bringen des in (ii) erhaltenen Zentrifugationsrückstands in einem Laemmli-Puffer, der 1 bis 5 % SDS enthält, Inkubation in diesem Puffer bei 100°C während 2 bis 10 Minuten und Zentrifugation bei 12.000 bis 15.000 g während 10 bis 15 Minuten bei 16 bis 22°C umfasst.

3. Verfahren nach Anspruch 1 oder Anspruch 2, **dadurch gekennzeichnet, dass** der Homogenisierungspuffer der Stufe (i) ein neutraler Puffer oder ein isotonischer Puffer ist.

4. Verfahren nach Anspruch 3, **dadurch gekennzeichnet, dass** der Homogenisierungspuffer unter Wasser und Glucose mit 5 % ausgewählt wird.

5. Verfahren nach einem der Ansprüche 1 bis 4, **dadurch gekennzeichnet, dass** in der Stufe (ii) vor der Zentrifugation wenigstens ein Inhibitor von Proteasen zugesetzt wird.

6. Verfahren nach einem der Ansprüche 1 bis 5, **dadurch gekennzeichnet, dass** die Zentrifugation in der Stufe (ii) nach Abscheidung der Suspension, die das PrPres enthält, auf einem Kissen aus Saccharose mit 6 bis 20 % durchgeführt wird.

7. Verfahren nach einem der Ansprüche 1 bis 6, **dadurch gekennzeichnet, dass** die während der Stufe (ii) der Extraktion zur Extraktion verwendete Lösung ein anionisches Detergens, geeignet, die Aggregation des PrPres zu begünstigen, und ein zwitterionisches Detergens wie zum Beispiel ein Sulfobetain in einem Verhältnis von 1:1 (V/V) enthält.

8. Verfahren nach einem der Ansprüche 1 bis 7, **dadurch gekennzeichnet, dass** die Zentrifugation in der Stufe der Extraktion (ii) nach Abscheidung der Suspension, die das PrPres enthält, auf einem Kissen, das ein Gemisch aus der Saccharose mit 6 bis 20 % und einem Sulfobetain enthält, durchgeführt wird.

9. Verfahren nach einem der Ansprüche 1 bis 8, **dadurch gekennzeichnet, dass** das Organ oder Gewebe unter der Milz und dem Gehirn ausgewählt ist.

10. Verfahren nach einem der Ansprüche 1 bis 9, **dadurch gekennzeichnet, dass** die Zentrifugation in der Stufe (ii) während 1 bis 2 Stunden bei einer Temperatur von 16 bis 22°C mit 25.000 bis 30.000 g durchgeführt wird.

11. Verfahren zur Durchmusterung von Substanzen, von denen angenommen wird, dass sie eine therapeutische Wirkung bei der Behandlung von subakuten, spongiformen, übertragbaren Encephalopathien [encephalopathies subaiguës spongiformes transmissibles (ESST)] haben, **dadurch gekennzeichnet, dass** es die folgenden Stufen umfasst:
a) Inoculation zum Zeitpunkt t_{A} wenigstens eines Labortiers, ausgewählt aus der Gruppe, bestehend aus Nagern, auf jedem geeigneten Weg mit einem übertragbaren nichtherkömmlichen Agens (ATNC);
b) Verabreichung an das Labortier auf jedem geeigneten Weg einer zu durchmusternden Substanz (Tiertest) oder eines Placebos (negative Tierkontrolle) in einem Zeitraum zwischen t_{A}-15 Tagen und t_{C}, das dem Zeitpunkt entspricht, an dem PrPres in der Milz des Labortiers maximal ist, oder in einem Zeitraum zwischen t_{B}, das dem Zeitpunkt der ersten Detektion von PrPres in der Milz des Labortiers entspricht, und t_{C}; wobei t_{B} zwischen t_{A} und t_{A}+15 liegt und t_{C} zwischen t_{A}+25 und t_{A}+30 liegt;
c) Töten der Tiere in einem Zeitraum zwischen t_{B} und t_{C} und Entnahme der Milz, wobei t_{A}, t_{B} und t_{C} in Tagen ausgedrückt werden.
d) Isolierung des PrPres aus jeder entnommenen Milz nach einem Verfahren zur Isolierung nach einem der Ansprüche 1 bis 10;
e) Halbquantitative Bestimmung des PrPres, das in der Stufe d) erhalten wurde, durch Detektion des PrPres durch jedes geeignete Verfahren, das ein spezifisches Signal liefert, gefolgt von einem Vergleich des erhaltenen Signals mit einer Standardskala von Verdünnungen einer positiven Kontrolle, die aus einem Gehirnhomogenisat eines Tiers im Endstadium der Krankheit besteht;
f) Auswahl der durchgemusterten Substanz als Kandidat zur Behandlung von subakuten, spongiformen, übertragbaren Encephalopathien, wenn der Wert für PrPres, der in der Milz des Testtiers in der Stufe e) erhalten wurde, um wenigstens einen Faktor 2 im Vergleich zu dem Wert verringert ist, der unter den selben Bedingungen mit dem Tier als negative Kontrolle erhalten wurde.

12. Verfahren zur Durchmusterung nach Anspruch 11, **dadurch gekennzeichnet, dass** das ATNC in Stufe a) in einem Puffer, der für den gewählten Verabreichungsweg angepasst ist, in Form eines Geweberohhomogenisats oder eines PrPres-Rückstands, erhalten durch zweckmäßige Zentrifugation, aus einem Geweberohhomogenisat, verabreicht wird.

13. Verfahren nach Anspruch 12, **dadurch gekennzeichnet, dass** das Geweberohhomogenisat ein Gehirnhomogenisat ist.

14. Verfahren zur Durchmusterung nach einem der Ansprüche 11 bis 13, **dadurch gekennzeichnet, dass** das ATNC in der Stufe a) auf intraperitonealem Weg mit einer Dosis, die einem ATNC-Inoculum entspricht und zwischen 0,001 % und 10 %, als Gewicht/Volumen, umfasst, wobei die LD₅₀ zwischen 10⁻³ und 10⁻⁷ ist.

15. Verfahren zur Durchmusterung nach einem der Ansprüche 11 bis 14, **dadurch gekennzeichnet, dass** das Verfahren zur Isolierung des PrPres in Stufe d) eine Abtrennung in einer einzigen Stufe umfasst.

16. Verfahren zur Durchmusterung nach einem der Ansprüche 11 bis 15, **dadurch gekennzeichnet, dass** das PrPres in Stufe e) durch Immunoassay detektiert wird.

17. Verfahren nach einem der Ansprüche 11 bis 16, **dadurch gekennzeichnet, dass** die Tötung in Stufe c) zum Zeitpunkt t_{C} durchgeführt wird.
